# EUROPÄISCHE PATENTSCHRIFT

(11) **EP 2 563 478 B1**
(45) Veröffentlichungstag und Bekanntmachung des Hinweises auf die Patenterteilung: **23.10.2013**
(21) Anmeldenummer: 12730779.1
(22) Anmeldetag: 04.05.2012
(51) Int. Cl.: A61K 35/74

(54) **VERFAHREN ZUR HERSTELLUNG EINES PHARMAZEUTISCH WIRKSAMEN EXTRAKTS AUS ARTHROSPIRA SPEC.**
PROCESS FOR THE PREPARATION OF A PHARMACEUTICALLY EFFECTIVE EXTRACT FROM ARTHROSPIRA SPEC.
PROCÉDÉ DE PRÉPARATION D'UN EXTRAIT PHARMACEUTIQUEMENT ACTIF D'ARTHROSPIRA SPEC.

(30) Priorität: 06.07.2011 DE 102011107307
(43) Veröffentlichungstag der Anmeldung: 06.03.2013
(73) Patentinhaber: Ocean Research & Development GmbH, 21465 Reinbek (DE)
(72) Erfinder: KEIMES, Jörg, 21465 Reinbek (DE); GÜNTHER, Patrick, 21521 Aumühle (DE)
(74) Vertreter: Lobemeier, Martin Landolf
(86) Internationale Anmeldenummer: PCT/DE2012/000455
(87) Internationale Veröffentlichungsnummer: WO 2013/004205

(56) Entgegenhaltungen:
- WO-A1-2006/047830
- WO-A2-2010/125490
- OZDEMIR GUVEN ET AL: "Antibacterial activity of volatile component and various extracts of Spirulina platensis.", PHYTOTHERAPY RESEARCH : PTR SEP 2004 LNKD- PUBMED:15478198, Bd. 18, Nr. 9, September 2004 (2004-09), Seiten 754-757, XP55033785, ISSN: 0951-418X
- CHIRASUWAN NATTAYAPORN ET AL: "Anti HSV-1 activity of sulphoquinovosyl diacylglycerol isolated from Spirulina platensis", SCIENCEASIA, Bd. 35, Nr. 2, Juni 2009 (2009-06), Seiten 137-141, XP002680721, ISSN: 1513-1874

## Beschreibung

Die Erfindung betrifft ein Verfahren zur Herstellung eines pharmazeutisch wirksamen Extrakts aus dem Cyanobakterium *Arthrospira spec..*

Es ist seit längerem bekannt, dass aquatische Organismen eine Vielzahl von molekularen Abwehrmechanismen gegen mikrobiellen, insbesondere bakteriellen und fungalen Befall entwickelt haben. Mit zunehmend ausgereiften molekularbiologischen Techniken sind diese Moleküle in neuerer Zeit auch in den Fokus von Untersuchungen für die medizinische Anwendung bei Erkrankungen von Mensch und Tier gerückt.

Insbesondere sind hier verschiedene Algen und Cyanobakterien zu nennen, die nicht nur reiner Forschungsgegenstand molekularer Abwehrmechanismen, sondern aufgrund ihrer Produktion von pharmazeutisch wirksamen Stoffen, unter anderem auch als Nahrungsergänzungsmittel ein bedeutender Wirtschaftsfaktor sind.

Beispielsweise ist vom Cyanobakterium *Arthrospira spec.* bekannt, dass dieses unter physiologischem Stress antimikrobiell wirksame Substanzen erzeugt, die durch geeignete Extraktionsschritte angereichert und zur Behandlung unterschiedlicher Beschwerden verwendet werden können. Ein derartiges Verfahren ist beispielsweise aus der WO 2006/047830 A1 bekannt.

Allerdings sind die Stressbedingungen unter denen die zur Produktion bestimmter Subtanzen verwendeten Organismen ein Maximum an den gewünschten Substanzen produzieren nur schwer zu ermitteln bzw. einzustellen, sodass häufig entweder nur wenig der gewünschten Substanzen produziert oder die Bedingungen zu harsch eingestellt werden, wodurch die Organismen absterben.

Aufgabe der Erfindung ist es daher, ein Verfahren zur Herstellung eines pharmazeutisch wirksamen Extrakts aus *Arthrospira spec.* zu schaffen, mit dem der natürlich vorkommende Gehalt an pharmazeutisch wirksamen Subtanzen in hohem Maße aus den Organismen extrahiert werden kann. Dabei soll der gewonnene Extrakt gute antimikrobielle, insbesondere bakterizide und fungizide Wirkung aufweisen.

Diese Aufgabe wird durch das Verfahren mit den Merkmalen von Anspruch 1 gelöst. Die Unteransprüche geben vorteilhafte Ausgestaltungen der Erfindung wieder.

Grundgedanke der Erfindung ist es, für den Zellaufschluss einen handelsüblichen Extruder zu verwenden, der ohnehin bei der *Arthrospira* verarbeitenden Industrie zur Herstellung von Produkten in Tablettenform vorhanden ist.

Speziell ist vorgesehen ein handelsüblich als Spirulina-Pulver zu erwerbendes Pulver aus getrockneten *Arthrospira*-Individuen, bevorzugt das Cyanobakterium *Arthrospira maxima, Arthrospira fusiformis* oder *Arthrospira platensis,* unter Zugabe von Wasser bei einer Temperatur zwischen jeweils einschließlich 130 °C und 160 °C und einem Druck zwischen jeweils einschließlich 8 MPa und 13 MPa (entspricht 80 bis 130 bar) zu extrudieren, sodass es am Matrizenkopf des Extruders nur kurzzeitig zu Temperaturen von höchstens 160 °C kommt. Die Auswahl eines für das erfindungsgemäße Verfahren geeigneten Extruders richtet sich also nach der Möglichkeit Wasser als Lösungsmittel zu verwenden wie auch nach den vorgenannten Parametern, die im Wesentlichen nicht überschritten, aber auch nicht unterschritten werden sollten.

In einem sich daran anschließenden Schritt werden die *Arthrospira*-Inhaltsstoffe mit einem organischen Lösungsmittel, bevorzugt Essigsäureethylester extrahiert. Besonders bevorzugt werden hierfür 10 ml Essigsäureethylester pro 1 g Extrudat verwendet, wobei die Extraktion äußerst bevorzugt über eine Zeitdauer von 24 h bei 28 °C erfolgt.

Um schließlich zu einem handelsfertigen Produkt zu gelangen, kann das bei der Extraktion verwendete Lösungsmittel entfernt, beispielsweise in einem Rotationsdampfer verdampft werden. Besonders bevorzugt wird der nach Entfernen des Lösungsmittels verbleibende Extrakt zum Entfärben und/oder zur Geruchsbeseitigung des Extrakts gefiltert, z.B. mit Aktivkohle, um schließlich einen wässrigen Extrakt zu erhalten, der zu therapeutischen oder prophylaktischen Zwecken im human- oder veterinärmedizinischen Bereich einsetzbar ist. Darüber hinaus ist es auch denkbar, dass der Extrakt im kosmetischen Bereich Anwendung findet.

Die Erfindung wird anhand eines besonders bevorzugt ausgestalteten Ausführungsbeispiels näher erläutert:

Nicht gentechnisch behandeltes Spirulina Pulver (*Spirulina platensis* bzw. *Arthrospira platensis*) wurde in einem Einwellenextruder der Firma Kahl vom Typ OEE8 zu Pellets verarbeitet.

Das verwendete *Arthrospira-*Pulver hatte die in Tabelle 1 dargestellten Eigenschaften:

**Tabelle 1**

| **Parameter** | **Methode/Gerät** | **Messwerte** |
|---|---|---|
| Farbe | visuell | dunkelgrün |
| Geruch | DAB 10 (V.3.1.6) | arteigen |
| Konsistenz | visuell | pulverförmig |
| Feuchte | DAB 10 (V.6.22.N2) Absolutbestimmer bei 105 °C | 3,92 g / 100 g |
| Protein | § 35 LMBG L 17.00-15 Kjeldahl-Bestimmer | 54,75 g / 100 g |
| Mineralstoffe | DAB 10 (V.3.2.16) Veraschung | 5,65 g / 100 g |
| Fett/Lipide | Mod. nach § 35 LMBG L 17.00-4; Extraktion nach Weibull / Stoldt | 5,6 g / 100 g |
| Algentoxine | HPLC, UV Detektion, Massenspektrometer | nicht nachweisbar |
| Chlorophyll | Jeffrey, SW and Humphrey, GF (1975) Biochem. Physiol. Pflanzen 167, 191-194, Spektralphotometer | 1,019 mg / 100 g |

Die Gesamtkeimzahl betrug 7 x 10⁴ Keime / g Pulver, wobei der Anteil an Hefen, Schimmelpilzen und coliformen Keimen jeweils geringer als 100 Zellen / g Pulver waren (*Escherichia coli* selbst war nicht nachweisbar).

Der für dieses Beispiel verwendete Extruder zeichnet sich durch eine hohe Belastbarkeit, die Möglichkeit der Arbeit mit hohem Druck (bis 150 Bar), hohes Leistungsniveau (bis 11kW), eine hydraulisch gesteuerte Matrize und die Zuführung von Flüssigkeiten in den laufenden Prozess aus. Insbesondere können über ein Touchpanel Druck, Umdrehungsgeschwindigkeit der Extruderwelle, Produkteintrag und die Zufuhr von Flüssigkeiten, hier: Wasser, gesteuert werden. Zwei Temperaturfühler ermöglichen die Beobachtung und mögliche Gegensteuerung durch Kühlung. Der Extruder nutzt in der Eindringtiefe verstellbare konisch zulaufende Schrauben, die die zweite Welle ersetzen.

Der besondere Vorteil der Extrusion ist die sehr kurze Temperaturbelastung des Mikroalgenpulvers von maximal 2 Minuten, abhängig von der eingestellten Verweildauer. Wie sich herausgestellt hat, ermöglicht das Zusammenspiel von Druck und Scherkräften trotz sehr kurzer Temperaturbelastung eine hohe Aufschlussrate. Speziell ist es beim verwendeten Extruder möglich, den Extruder mit Kühlflüssigkeit zu kühlen, sodass über den gesamten Prozessablauf relativ niedrige Temperaturen aufrecht erhalten werden können.

In einem ersten Schritt wird das *Arthrospira*-Pulver über eine Dosierschnecke in den Extruder gefördert. Nach einem kurzen Knetweg wird das Pulver durch die Zufuhr von Wasser im Bereich von jeweils einschließlich 2 l/h bis 4 l/h befeuchtet. Erst jetzt beginnen die Vermischung mit Wasser und das Scheren des *Arthrospira*-Pulver/Wasser-Gemischs.

Der Durchsatz an Pulver während des laufenden Prozesses beträgt bevorzugt etwa 40 kg/h, wobei die bevorzugt verwendete Standardwelle (Nr. 3195-4456) mit 300 U/min betrieben wird.

Am Ende der Schnecke verschließt eine Matrize den Extruder, die es dem Gemisch aus Alge und Wasser nur durch Bohrungen und/oder Schlitze vorbestimmter Anzahl und Form ermöglicht, aus dem Exruder auszutreten. Bevorzugt wird eine Matrize mit 14 Bohrungen mit einem Bohrungsdurchmesser von jeweils 3,0 mm verwendet. Zwangsläufig kommt es zu einem Zusammenspiel von Druck und Scherung, bis das Extrudat sich durch die Bohrungen bzw. Schlitze drückt. Bei dem Austreten des Extrudats aus dem Extruder durch die Matrize kommt es zur Expansion des Extrudats aufgrund der Verdampfung des Lösungsmittels und des plötzlichen Temperaturabfalls.

Bevorzugt schneidet ein hinter der Matrize angeordneter, rotierender Messerkopf das Extrudat in Pellets, die beim Herunterfallen in einen Auffangbehälter durch die vergrößerte Oberfläche schnell trocknen und abkühlen. An der Matrize kann es dabei kurzfristig zu Temperaturen von bis zu 160 °C kommen.

Zur Extraktion der pharmazeutisch wirksamen Arthrospira-Inhaltsstoffe werden jeweils 10 ml Ethylacetat zu 1 g des extrudierten *Arthrospira*-Pulvers gegeben und 24 h lang bei 28 °C inkubiert. Die Lösung wird nach Inkubation bevorzugt durch einen Faltenfilter filtriert und das Lösungsmittel in einem Rotationsverdampfer verdampft. Das Filtrat wird darauf bevorzugt in 1 ml Ethanol aufgenommen und durch einen Sterilfilter filtriert.

Besonders bevorzugt kann der Extrakt zur Entfärbung und/oder Geruchsreduktion weiter filtriert, beispielsweise über eine Aktivkohlesäule gegeben werden. Als Resultat erhält man eine klare Lösung mit einem arteigenen Geruch.

Der nach dem bevorzugt dargestellten Verfahren hergestellte Extrakt weist eine hohe antimikrobielle, insbesondere antibakterielle und antifungale Aktivität auf. Als Maß für die Bioaktivität wurde die Chitinase-Aktivität des Extrakts gemessen.

So konnte in einem Extrakt aus Spirulina Powder High Performance, das nicht extrudiert, aber ansonsten wie oben beschrieben behandelt wurde, eine Chitinaseaktivität von 5,5 U/g nachgewiesen werden.

Demgegenüber zeigte eine Messung der Chitinase-Aktivität des nach dem erfindungsgemäßen Verfahren mit Extrusion behandelten Spirulina Powder High Performance durch eine gegenüber dem unbehandelten Pulver um das annähernd 3,5-fache gesteigerte Aktivität von von 19,1 U/g.

Die Wirkungen des Algenextraktes gegen Pilze, Bakterien und Viren wurden im ersten Schritt *in-vitro* durchgeführt, d.h. klassische Hemmhoftest gegen Pilze und Bakterien. Insbesondere wurden Bioaktivitätstests (u.a. Hemmhoftests) mit *Propionibakterium acnes, Bacillus subtilis, Escherichia coli, Trichophytum rubrum, Trichophytum mentagrophytes* und Herpes durchgeführt. Die antivirale Schutzwirkung gegen Herpes-Viren (HHV-1) wurde anhand von Vero-Zellen geprüft. Nach erfolgreich positiven in-vitro Tests wurden weiter Therapierhebung und Fallbeobachtungen an Patienten durchgeführt, um den klinischen Effekt zu überprüfen.

Das Ergebnis zeigt eine gegenüber der antimikrobiellen Aktivität eines ohne Extrudieren des Ausgangsmaterials hergestellten *Arthrospira-Extrakts* (Extrakt 1) gesteigerte Wirkung der antimikrobiellen Aktivität des erfindungsgemäß hergestellten Extrakts (Extrakt 2). Zusätzlich wurden die für *Arthrospira platensis* erhaltenen Messwerte (Extrakt 1 und Extrakt 2) mit der antimikrobiellen Aktivität eines mit dem Verfahren nach der Erfindung hergestellten Extraktes aus *Arthrospira maxima* (Extrakt 3) verglichen.

Die in Tabelle 2 wiedergegebenen Versuchergebnisse zeigen für *Propionibakterium acnes, Bacillus subtilis, Escherichia coli, Trichophytum rubrum, Trichophytum mentagrophytes* die Bioaktivität des jeweiligen Extrakts anhand der inhibitorischen Wirkung des Extrakts in %. Mit 0 % wurden alle Aktivitäten mit einer inhibitorischen Wirkung von 30 % und/oder geringer bezeichnet. Für Herpes-Viren konnte eine inhibitorische Wirkung für Extraktlösungen >2 % nachgewiesen werden.

**Tabelle 2.**

| | *P. acnes* | *B. subtilis* | *E. coli* | *T. rubrum* | *T. mentagrophytes* |
|---|---|---|---|---|---|
| Extrakt 1 - 0,1% | 0 | 0 | 0 | 0 | 0 |
| Extrakt 1 - 1% | 98 | 33 | 0 | 43 | 40 |
| Extrakt 1 - 5% | 100 | 54 | 0 | 52 | 51 |
| Extrakt 2 - 0,1% | 0 | 0 | 0 | 0 | 0 |
| Extrakt 2 - 1% | 81 | 37 | 0 | 59 | 54 |
| Extrakt 2 - 5 % | 100 | 56 | 45 | 73 | 65 |
| Extrakt 3 - 0,1% | 0 | 0 | 0 | 0 | 0 |
| Extrakt 3 - 1% | 87 | 35 | 0 | 51 | 45 |
| Extrakt 3 - 5 % | 93 | 75 | 55 | 63 | 57 |

In einem weiteren Versuch wurde die Hemmung weiterer Erreger bei einem Einsatz von 0,5 % des gelösten, erfindungsgemäß hergestellten Extrakts untersucht (Tabelle 3):

**Tabelle 3**

| Erreger | Prozentuale Hemmung |
|---|---|
| *Propionibacterium acnes* | 100% |
| *Staphylococcus epidermis* | 100% |
| *Staphylococcus aureus* | 100% |
| *Pseudomonas aeruginosa* | 90% |
| *Aspergillus niger* | 80% |
| *Dermabacter* | 90% |
| *Brevibacter* | 97% |
| *Trichoderma* Arten | 100% |

Das erfindungsgemäße Verfahren ermöglicht einen Aufschluss von *Arthrospira-*Pulver zur Herstellung eines pharmazeutisch wirksamen *Arthrospira*-Extraktes mit gesteigerter antimikrobieller Aktivität. Das Verfahren vermeidet komplizierte und aufwändige Methoden zur gesteigerten Produktion pharmazeutisch aktiver Substanzen durch den Organismus selbst, sondern erhöht die Ausbeute dieser Substanzen in einem Extrakt herkömmlich oder auch unter physiologischem Stress gezüchteter Arthrospira-Cyanobakterien gegenüber mit herkömmlichen Verfahren hergestellten Extrakten.

Speziell eignet sich der erfindungsgemäße Extrakt zur Verwendung gegen Krankheiten, die insbesondere durch *Propionibacterium acnes, Staphylococcus epidermis, Staphylococcus aureus, Pseudomonas aeruginosa, Aspergillus niger, Dermabacter, Brevibacter* und *Trichoderma* Arten, einzeln oder in Kombination, hervorgerufen werden. Diese Krankheiten sind insbesondere Hautkrankheiten, beispielsweise Akne, Herpes (insbesondere Typ I), Blepharitis, Endophtalmitis.

Darüber hinaus konnte in Fallstudien auch eine Wirkung des erfindungsgemäß hergestellten Extrakts gegen Viren aus der Gruppe der Humanen Papillomaviren (HPV) festgestellt werden.

## Patentansprüche

1. Verfahren zur Herstellung eines pharmazeutisch wirksamen Extrakts aus dem Cyanobakterium *Arthrospira spec.* mit den Schritten
- Extrudieren eines *Arthrospira spec.* enthaltenden Pulvers unter Zugabe von Wasser;
- Extrahieren des Extrudats mit einem organischen Lösungsmittel; und
- Bereitstellen des Extrakts als pharmazeutisch wirksame Zusammensetzung.

2. Verfahren nach Anspruch 1, **dadurch gekennzeichnet, dass** das Extrudieren bei einer Temperatur zwischen jeweils einschließlich 130 °C und 160 °C und einem Druck zwischen jeweils einschließlich 8 MPa und 13 MPa erfolgt.

3. Verfahren nach einem der vorhergehenden Ansprüche, **dadurch gekennzeichnet, dass** das organische Lösungsmittel Essigsäureethylester ist.

4. Verfahren nach einem der vorhergehenden Ansprüche, **dadurch gekennzeichnet, dass** das Extrahieren bei 28 °C für 24 h erfolgt.

5. Verfahren nach einem der vorhergehenden Ansprüche, **gekennzeichnet durch** die sich an das Extrahieren anschließenden Schritte:
- Entfernen des Lösungsmittels;
- Filtrieren des Extrakts zum Entfärben und/oder zur Geruchsbeseitigung des Extrakts; und
- Bereitstellen des filtrierten Extrakts als pharmazeutisch wirksame Zusammensetzung.

6. Verfahren nach Anspruch 5, **dadurch gekennzeichnet, dass** das filtrierte Extrakt in Ethanol aufgenommen und/oder durch einen Sterilfilter filtriert wird.

7. Verfahren nach einem der vorhergehenden Ansprüche, **dadurch gekennzeichnet, dass** eine wässrige Lösung von Arthrospira spec. enthaltendem Pulver extrudiert wird.

8. Verfahren nach einem der vorhergehenden Ansprüche, **dadurch gekennzeichnet, dass** dem Pulver beim Extrudieren mit einem Durchsatz von 40 kg Pulver pro Stunde 21 bis 41 Wasser pro Stunde zugeführt wird.

9. Verfahren nach einem der vorhergehenden Ansprüche, **dadurch gekennzeichnet, dass** das Extrahieren mit 10 ml Essigsäureethylester pro 1 g Extrudat erfolgt.

10. Verfahren nach einem der vorhergehenden Ansprüche, **dadurch gekennzeichnet, dass** das Cyanobakterium *Arthrospira maxima, Arthrospira fusiformis* oder *Arthrospira platensis* ist.

## Claims

1. A process for the preparation of a pharmaceutically effective extract from the cyanobacterium *Arthrospira spec.* with the steps
- extrusion of a powder containing *Arthrospira spec.* with the addition of water;
- extraction of the extrudate with an organic solvent; and
- provision of the extract as a pharmaceutically effective composition.

2. The process according to Claim 1, **characterized in that** extrusion takes place at a temperature in each case inclusively between 130°C and 160°C and a pressure between in each case inclusively 8MPa and 13MPa.

3. The process according to one of the preceding claims, **characterized in that** the organic solvent is ethyl acetate.

4. The process according to one of the preceding claims, **characterized in that** extrusion takes place at 28°C for 24h.

5. The process according to one of the preceding claims, **characterized by** the steps following the extrusion:
- removing the solvent;
- filtration of the extract for discolouring and/or for deodorizing the extract; and
- provision of the filtered extract as a pharmaceutically effective composition.

6. The process according to Claim 5, **characterized in that** the filtered extract is taken up ethanol and/or is filtered through a sterile filter.

7. The process according to one of the preceding claims, **characterized in that** an aqueous solution of powder containing Arthrospira spec. is extruded.

8. The process according to one of the preceding claims, **characterized in that** during extrusion between 2L and 4L of water is added per hour to the powder at a throughput of 40kg of powder per hour.

9. The process according to one of the preceding claims, **characterized in that** extrusion takes place using 10mL of ethyl acetate per 1g of extrudate.

10. The process according to one of the preceding claims, **characterized in that** the cyanobacterium is *Arthrospira maxima, Arthrospira fusiformis* or *Arthrospira platensis.*

## Revendications

1. Procédé de préparation d'un extrait pharmaceutiquement actif de la cyanobactérie Arthrospira spec. comprenant les étapes suivantes
- Extrusion d'une poudre contenant *Arthrospira spec.* tout en ajoutant de l'eau ;
- Obtention d'un extrait à partir de l'extrudat, par extaction à l'aide d'un solvant organique ; et
- Préparation de l'extrait sous forme de composition pharmaceutiquement active.

2. Procédé selon la revendication 1 **caractérisé en ce que** l'extrusion doit avoir lieu à une température comprise exclusivement entre 130 °C et 160 °C et une pression comprise exclusivement entre 8 MPa et 13 MPa.

3. Procédé selon l'une des revendications précédentes, **caractérisé en ce que** le solvant organique est de l'acétate d'éthyle.

4. Procédé selon l'une des revendications précédentes, **caractérisé en ce que** l'extraction est effectuée à 28 °C pendant 24h.

5. Procédé selon l'une des revendications précédentes, **caractérisé par** les étapes suivantes de l'extraction :
- Elimination du solvant ;
- Filtration de l'extrait pour obtenir une décoloration et/ou une désodorisation de l'extrait ; et
- Préparation de l'extrait filtré sous forme de composition pharmaceutiquement active.

6. Procédé selon la revendication 5, **caractérisé en ce que** l'extrait filtré est repris dans de l'éthanol et/ou filtré par un filtre stérile.

7. Procédé selon l'une des revendications précédentes, **caractérisé en ce que** l'on extrude une solution aqueuse d'Arthrospira spec. contenant de la poudre.

8. Procédé selon l'une des revendications précédentes, **caractérisé en ce que** l'on alimente la poudre de l'extrusion à un débit de 40 kg de poudre/heure pour 2 à 4 d'eau/heure.

9. Procédé selon l'une des revendications précédentes, **caractérisé en ce que** l'extraction s'effectue l'aide de 10 ml d'acétate d'éthyle pour 1 g d'extrudat.

10. Procédé selon l'une des revendications précédentes, **caractérisé en ce que** la cyanobactérie soit de type *Arthrospira maxima, Arthrospira fusiformis* ou *Arthrospira platensis.*
